# EUROPEAN PATENT APPLICATION

(11) **EP 4 846 860 A1**
(43) Date of publication of application: **02.12.2026**
(21) Application number: 25744979.3
(22) Date of filing: 08.01.2025
(51) Int. Cl.: A23L 33/10, A61K 31/661, A61P 43/00, C12N 15/00

(54) **DNA METHYLATION PROMOTER, ORAL COMPOSITION FOR PROMOTING DNA METHYLATION, AND USE OF COMPOUND**

(30) Priority: 25.01.2024 JP 2024009152
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO, Takehiko, Fukuoka-shi, Fukuoka 813-0043 (JP); MAWATARI, Shiro, Fukuoka-shi, Fukuoka 813-0016 (JP); NIWASE, Shamim, Fukuoka-shi, Fukuoka 810-0054 (JP); HONSHO, Masanori, Fukuoka-shi, Fukuoka 819-0395 (JP); OKAUCHI, Tatsuo, Kitakyushu-shi, Fukuoka 804-8550 (JP)
(74) Representative: V.O.
(86) International application number: PCT/JP2025/000250
(87) International publication number: WO 2025/158896

(57) **Abstract**

A DNA methylation promoter includes a compound of Formula (I), a racemate of the compound, or a salt of the compound or the racemate, 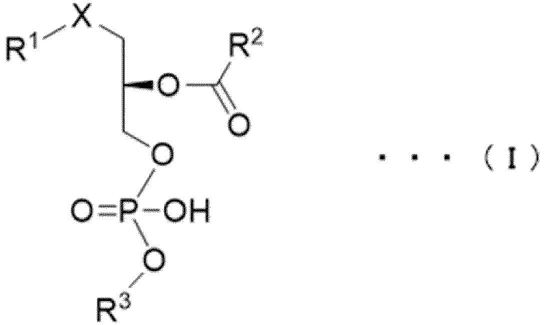 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 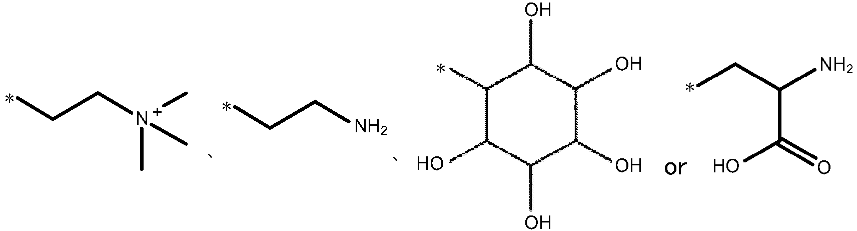 optionally having one or more substituents, in which * is an oxygen atom bound to R³).

## Description

### Technical Field

The present disclosure relates to a DNA methylation promoter, an oral composition for promoting DNA methylation, and use of a compound.

### Background Art

Methylation of a carbon at the 5-position of cytosine (5-cytosine) in DNA is a physiological DNA modification mechanism. Most DNA methylation modifications in mammals occur in sequences in which cytosine and guanine are consecutive (CpG sequences). Methylation of 5-cytosine in DNA of mammals plays an important role in many biological processes, such as genomic imprinting, cell fate determination, formation of chromatin structure, regulation of gene expression, and suppression of movement of retrotransposons (see Non Patent Literature 1 to 3).

In addition, it has been shown that an increase in autonomous transposition of retrotransposons caused by exposure to chemical substances during pregnancy or by aging may cause changes in functions of other genes (see Non Patent Literature 4), and causes chronic inflammation due to aging (see Non Patent Literature 5).

As described above, it is presumed that a decrease in methylation of genomic DNA is associated with various diseases, and Patent Literature 1 discloses an extract of *Rosa rugosa* and Patent Literature 2 discloses an extract of *Lagenaria siceraria var. hispida* fruit, the extracts having an inhibitory effect on demethylation of DNA induced by ultraviolet irradiation and a remethylation promoting effect on demethylation of DNA.

### Citation List

### Patent Literature

Patent Literature 1: Unexamined Japanese Patent Application Publication No. 2019-77621
Patent Literature 2: Unexamined Japanese Patent Application Publication No. 2019-77622

### Non Patent Literature

Non Patent Literature 1: Rudolf Jaenisch and Adrian Bird, "Epigenetic regulation of gene expression: how the genome integrates intrinsic and environmental signals", Nature Genetics, 2003, 33, 245-254
Non Patent Literature 2: Zachary D. Smith and Alexander Meissner, "DNA methylation: roles in mammalian development", Nature Reviews Genetics, 2013, 14, 204-220
Non Patent Literature 3: Keiko Nohara, and five others, "Gestational arsenic exposure induces site-specific DNA hypomethylation in active retrotransposon subfamilies in offspring sperm in mice", Epigenetics & Chromatin, 2020, 13, Article No. 53
Non Patent Literature 4: Christopher H. Switzer, and four others, "NOS2 and S-nitrosothiol signaling induces DNA hypomethylation and LINE-1 retrotransposon expression", Proc Natl Acad Sci USA, 2022, 119, 21, e2200022119
Non Patent Literature 5: Marco De Cecco, and 20 others, "L1 drives IFN in senescent cells and promotes age-associated inflammation", Nature, 2019, 566, 73-78

### Summary of Invention

### Technical Problem

In Patent Literature 1 and Patent Literature 2, an effective component having an inhibitory effect on demethylation of DNA and a remethylation promoting effect on demethylation of DNA in an extract of *Rosa rugosa* or an extract of *Lagenaria siceraria var. hispida* fruit has not been identified. In development of pharmaceuticals and the like, it is desirable that an effective component is known.

In view of the above circumstances, an objective of the present disclosure is to provide a DNA methylation promoter, an oral composition for promoting DNA methylation, and use of a compound that are capable of promoting methylation of genomic DNA.

### Solution to Problem

A DNA methylation promoter according to a first aspect of the present disclosure includes:
a compound of Formula (I), a racemate of the compound, or a salt of the compound or the racemate, 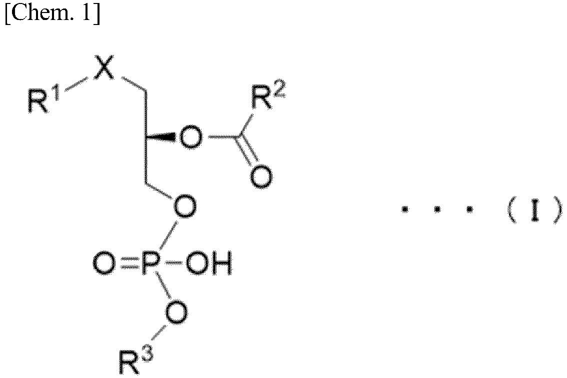 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 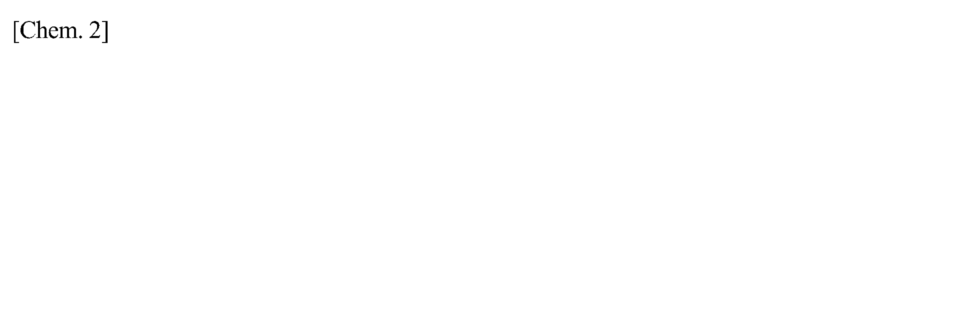 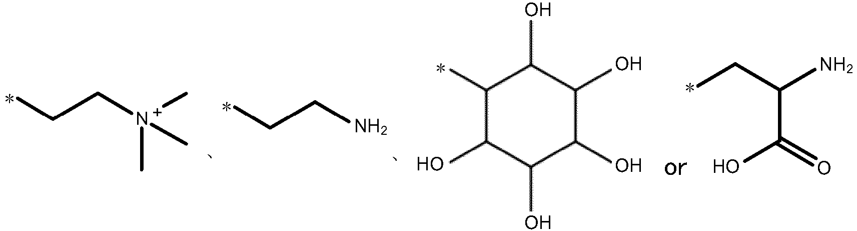 optionally having one or more substituents, in which * is an oxygen atom bound to R³).

An oral composition for promoting DNA methylation according to a second aspect of the present disclosure includes:
a compound of Formula (I), a racemate of the compound, or a salt of the compound or the racemate, 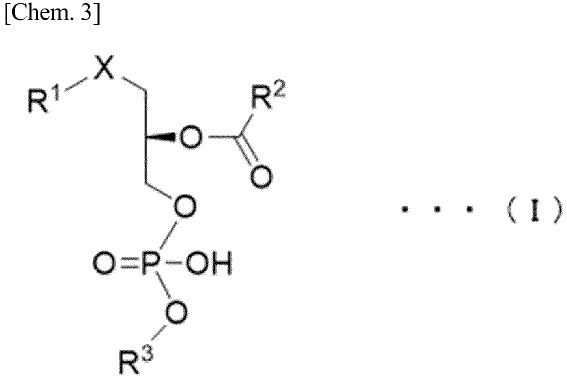 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 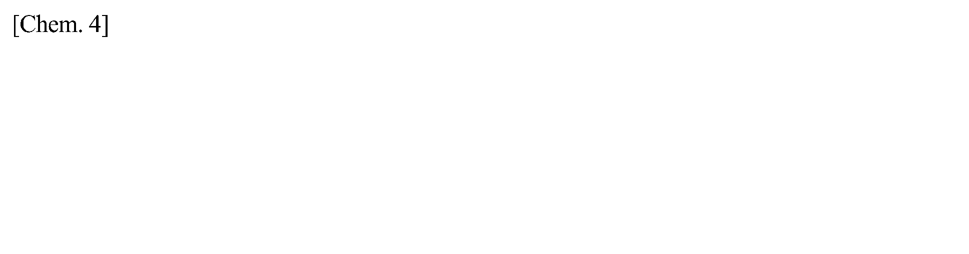 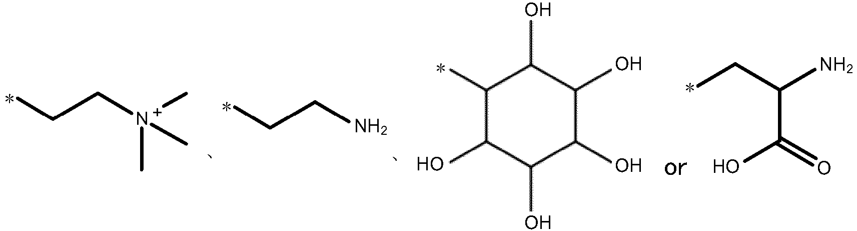 optionally having one or more substituents, in which * is an oxygen atom bound to R³).

X may represent an oxygen atom, and R¹ may represent an alkyl group having 18 carbon atoms.

R², when defined as R²COOH, may represent a monovalent group corresponding to arachidonic acid, oleic acid, or palmitic acid.

Use of a compound according to a third aspect of the present disclosure is
use of a compound of Formula (I), a racemate of the compound, or a salt of the compound or the racemate, for producing a DNA methylation promoter or an oral composition for promoting DNA methylation, 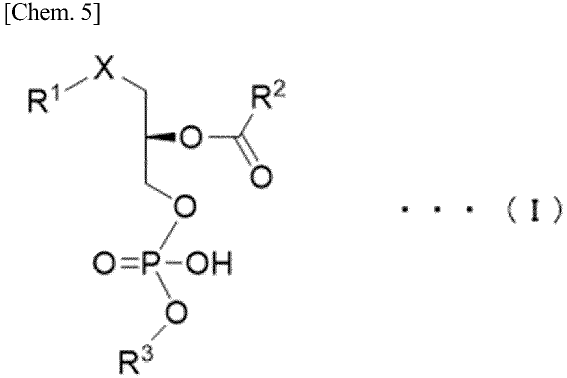 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 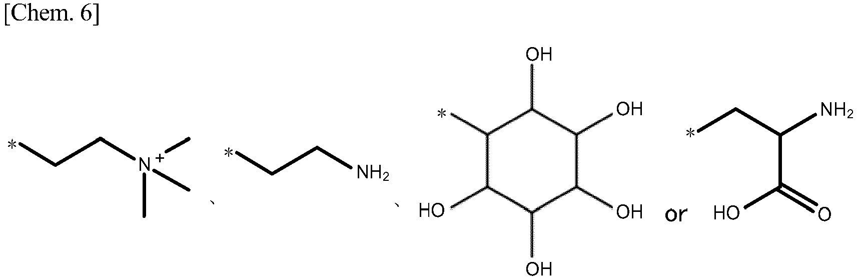 optionally having one or more substituents, in which * is an oxygen atom bound to R³

### Advantageous Effects of Invention

According to the present disclosure, methylation of genomic DNA can be promoted.

### Brief Description of Drawings

FIG. 1 is a diagram showing fluorescent staining images according to Test Example 1;
FIG. 2 is a diagram showing fluorescence intensity based on the fluorescent staining images shown in FIG. 1;
FIG. 3 is a diagram showing fluorescent staining images according to Test Example 2;
FIG. 4 is a diagram showing an anti-5-methylcytosine (5-mC) antibody positivity rates based on the fluorescent staining images shown in FIG. 3; and
FIG. 5 is a diagram showing the anti-5-mC antibody positivity rates according to Test Example 3.

### Description of Embodiments

Embodiments according to the present disclosure are described below with reference to the drawings. In this regard, the present disclosure is not limited by the below-described embodiments or the drawings. Note that, in the below-described embodiments, the expression, "have", "include", or "contain" encompasses the meaning of "composed of" or "be constituted by".

### Embodiments

A DNA methylation promoter according to the present embodiment includes a compound of Formula (I), a racemate of the compound, or a salt of the compound or the racemate.

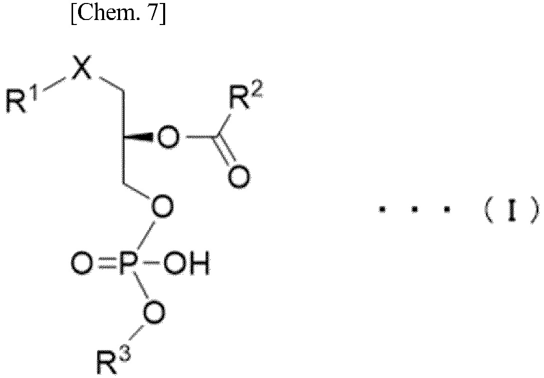

In Formula (I), X represents a carbon atom or an oxygen atom. X preferably represents an oxygen atom. R¹ represents a saturated aliphatic hydrocarbon group optionally containing a ring structure. The number of carbon atoms of the saturated aliphatic hydrocarbon group is not limited. The number of carbon atoms of the saturated aliphatic hydrocarbon group may be, for example, 10 to 30, 10 to 25, or 10 to 20. The number of carbon atoms constituting the ring structure is, for example, 3 to 6. The ring structure may be contained as a cycloalkylene group in R¹, or as a cycloalkyl group in R¹. The number of carbon atoms constituting the ring structure is preferably 3. The saturated aliphatic hydrocarbon group may contain the ring structure, as a monovalent group, at the terminus of R¹. R¹ preferably represents an alkyl group having 18 carbon atoms.

R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms. For example, R², when defined as R²COOH, represents a monovalent group corresponding to ω-3 fatty acids, ω-6 fatty acids, ω-7 fatty acids, ω-9 fatty acids, and ω-10 fatty acids. R², when defined as R²COOH, preferably represents a monovalent group corresponding to arachidonic acid, docosahexaenoic acid, oleic acid, palmitic acid, eicosapentaenoic acid, or linoleic acid. R², when defined as R²COOH, preferably represents a monovalent group corresponding to arachidonic acid, oleic acid, or palmitic acid.

Each carbon atom of a glycerol backbone in Formula (I) may have one or more substituents. Examples of the substituent of the carbon atom of the glycerol backbone include hydroxy, halogen, aryl, C1-C4 alkyl, and C1-C4 alkoxy. R³ is 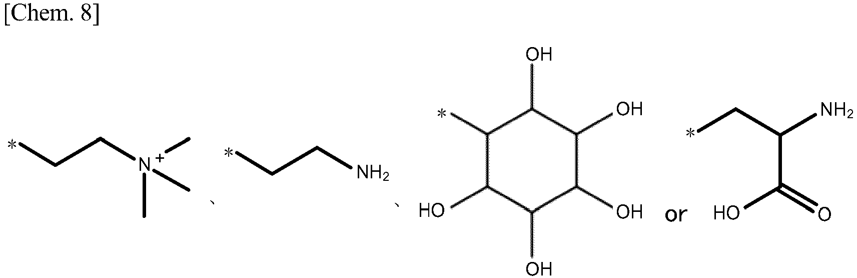 optionally having one or more substituents, in which * is an oxygen atom bound to R³. Examples of the substituent of R³ include hydroxy, halogen, aryl, C1-C4 alkyl, and C1-C4 alkoxy.

The salt of the compound of Formula (I) above and the salt of the racemate of the compound are not limited provided that the salts are pharmacologically acceptable salts, and may be either an acidic salt or a basic salt. Examples of the salts include alkali metal salts such as lithium salt, sodium salt, and potassium salt; alkaline earth metal salts such as magnesium salt and calcium salt; inorganic acid salts such as hydrochloride, hydrobromide, sulfate, nitrate, oxalate, and phosphate; and organic acid salts such as acetate, propionate, hexanoate, cyclopentane propionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, glucoheptanoate, 3-phenylpropionate, trimethylacetate, tertiary butyl acetate, lauryl sulfate, gluconate, glutamate, hydroxynaphthoate, salicylate, stearate, trifluoroacetate (TFA), maleate, and muconate.

For example, a DNA methylation promoter according to the present embodiment includes a compound of Formula (II), a racemate of the compound, or a salt of the compound or the racemate. X, R¹, and R² in Formula (II) are the same as X, R¹, and R² in Formula (I) described above.

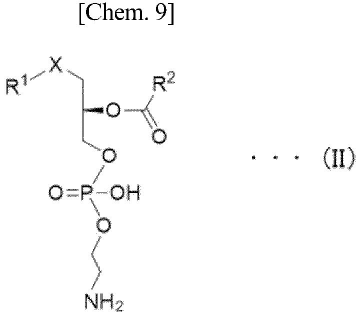

A DNA methylation promoter according to the present embodiment preferably includes a compound of any of Formula (i), Formula (ii), and Formula (iii), a racemate of the compound, or a salt of the compound or the racemate.

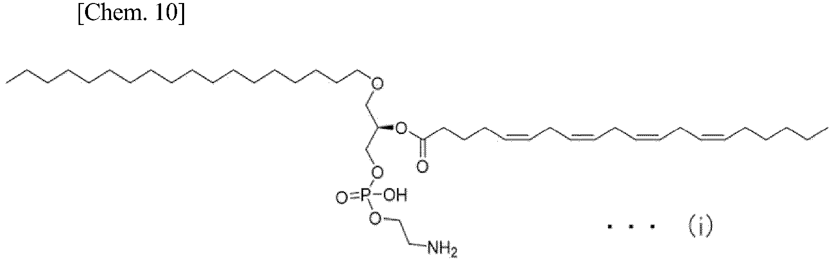

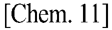

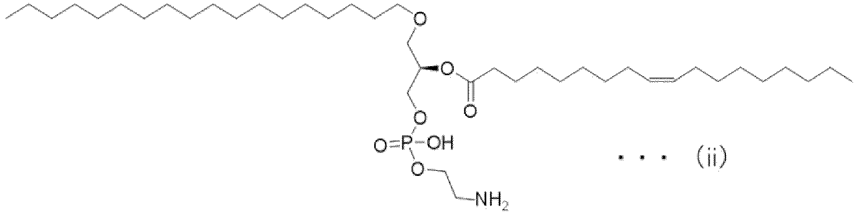

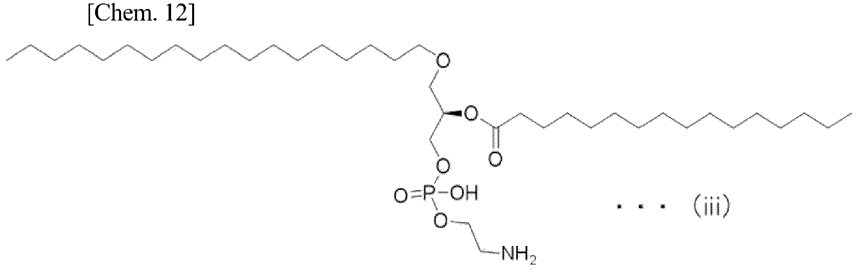

The compound according to the present embodiment can be synthesized by a known method. For example, R²COOH is reacted with tert-butyl(2-((tert-butoxy((R)-2-hydroxy-3-(octadecyloxy)propoxy)phosphoryl)oxy)ethyl )carbamate using a condensing agent. The resulting product is deprotected by allowing a strong acid such as trifluoroacetic acid to act on the product, to obtain a compound according to the present embodiment. The condensing agent is not limited, and may be, for example, 1-(3-dimethylaminopropyl)-3-ethylcarbodiimide (EDC) hydrochloride, dicyclohexylcarbodiimide (DCC), HATU, HBTU, TATU, TBTU, and diphenylphosphoryl azide (DPPA). For promoting the dehydration condensation reaction by the condensing agent, a nucleophile such as dimethylaminopyridine (DMAP) may be used.

The DNA methylation promoter according to the present embodiment contains the compound of Formula (I) above, the racemate of the compound, or the salt of the compound or the racemate (hereinafter also referred to as "compound or the like"), as effective components, and may also contain other pharmacologically acceptable components. Specific examples of the other pharmacologically acceptable components include excipients, lubricants, binders, disintegrators, solvents, solubilizers, suspending agents, tonicity agents, buffers, soothing agents, and the like. An additive such as antiseptic agents, antioxidants, coloring agents, or sweeteners may also be optionally added to the DNA methylation promoter.

Examples of the excipients include lactose, sucrose, D-mannitol, starch, crystalline cellulose, and light anhydrous silicic acid. Examples of the lubricants include magnesium stearate, calcium stearate, talc, and colloidal silica. Examples of the binders include crystalline cellulose, sucrose, D-mannitol, dextrin, hydroxypropylcellulose, hydroxypropylmethyl cellulose, and polyvinylpyrrolidone. Examples of the disintegrators include starch, carboxymethylcellulose, carboxymethylcellulose calcium, croscarmellose sodium, and carboxymethylstarch sodium.

Examples of the solvents include water for injection, alcohol, propylene glycol, and macrogol. Examples of the solubilizers include polyethylene glycol, propylene glycol, D-mannitol, benzyl benzoate, ethanol, trisaminomethane, cholesterol, triethanolamine, sodium carbonate, and sodium citrate. The suspending agents are a surfactant, a hydrophilic polymer, or the like, and the examples of the suspending agents include stearyl triethanolamine, sodium lauryl sulfate, lauryl aminopropionic acid, lecithin, benzalkonium chloride, benzethonium chloride, glycerin monostearate, polyvinyl alcohol, polyvinylpyrrolidone, sodium carboxymethylcellulose, methylcellulose, hydroxymethylcellulose, hydroxyethylcellulose, and hydroxypropylcellulose.

Examples of the tonicity agents include sodium chloride, glycerin, and D-mannitol. Examples of the buffers include buffer solutions such as phosphate, acetate, carbonate, and citrate. Examples of the soothing agents include benzyl alcohol. Examples of the antiseptic agents include paraoxybenzoic acid esters, chlorobutanol, benzyl alcohol, phenethyl alcohol, dehydroacetic acid, and sorbic acid. Examples of the antioxidants include sulfite and ascorbic acid.

The DNA methylation promoter according to the present embodiment promotes methylation of genomic DNA, as shown in the following Examples, compared with the level prior to administration of the DNA methylation promoter.

Since the DNA methylation promoter according to the present embodiment promotes methylation of genomic DNA, the DNA methylation promoter is useful for prevention and treatment of symptoms or diseases caused by a decrease or suppression of methylation of genomic DNA, or by a decrease in methylation-modified genomic DNA.

The DNA methylation promoter is produced by a known method, and contains the aforementioned compound or the like as an effective component. The DNA methylation promoter contains, for example, from 0.1% to 99% by weight, from 1% to 50% by weight, and preferably from 1% to 20% by weight of the compound or the like as an effective component.

The DNA methylation promoter according to the present embodiment is administered to humans or animals excluding humans. Preferred examples of the animals include mammals, more specifically, dogs, cats, cows, pigs, horses, sheep, and deer. The administration route of the DNA methylation promoter for humans or the like is not limited. The DNA methylation promoter is preferably used as an external preparation, an injection solution, or an oral preparation.

The DNA methylation promoter is provided in the form of liquids, pills, granules, fine granules, powders, tablets, capsules, or the like.

The dose of the DNA methylation promoter is appropriately determined based on the age, body weight, symptoms, and the like of the human or other subject to whom the DNA methylation promoter is administered. The DNA methylation promoter is administered in such an amount that the compound or the like is contained therein in an effective amount. An effective amount is the amount of the compound or the like necessary to promote the methylation of genomic DNA.

As described in detail above, according to the DNA methylation promoter of the present embodiment, methylation of genomic DNA can be promoted.

In another embodiment, an oral composition for promoting DNA methylation including the aforementioned compound or the like is provided. Specific examples of the oral composition for promoting DNA methylation include supplements, food compositions, foods and beverages, functional foods, and food additives. The DNA methylation promoter may be used as supplements, or may be added to foods and beverages and functional foods.

The forms of the supplements are not limited, and may be any of tablets, powders, granules, capsules, sugar-coated tablets, film formulations, troches, chewable formulations, solutions, emulsions, suspensions, and the like. The supplements may include any components that are normally used for supplements.

"Functional food" means a food or beverage consumed for the purpose of maintaining health, and examples of the functional foods include: foods for specified health uses, foods with function claims, and foods with nutrient function claims that are foods with health claims; health foods; dietary supplements; and the like. The functional foods are preferably foods for specified health uses or foods with nutrient function claims that are foods with health claims. In cases of commercialization as a functional food, the oral composition for promoting DNA methylation may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, flavors, and the like.

The functional food may be either a food or beverage, and is not limited as long as the functional food can be orally taken. Examples of the forms of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablet confectionery, chewing gums, and jellies. Examples of the processed cereal products include bread, cooked rice, and biscuits. Examples of the kneaded products include sausage, ham, and *kamaboko*, which is steamed fish cake made from whitefish paste. Examples of the dairy products include butter and yogurt.

The oral composition for promoting DNA methylation may be added to foods as food additives. In such a case, examples of the food additives may include pastes, gel-like agents, powders, liquids, suspensions, emulsions, and granules, from the viewpoint of easily adding the food additive to foods.

The oral composition for promoting DNA methylation may contain water, vitamins, minerals, organic acids, organic bases, fruit juices, flavors, functional components, food additives, and the like, as long as an action of promoting methylation of genomic DNA is maintained. The oral composition for promoting DNA methylation may be produced by a known method by adding components other than the compound or the like as necessary.

The oral composition for promoting DNA methylation may be divided into one or more containers such that the daily intake is in accordance with the above-described amount of intake. In such a case, the oral composition for promoting DNA methylation is preferably contained in each container in an amount corresponding to the daily intake.

From the viewpoint of the fact that the oral composition for promoting DNA methylation is used for promoting methylation of genomic DNA, the oral composition for promoting DNA methylation is provided in a manner that allows the oral composition to be distinguishable from other products. For example, at least one of the product packaging, instructions, and advertisements for the oral composition for promoting DNA methylation indicates that the oral composition has an action of promoting methylation of genomic DNA.

When the oral composition for promoting DNA methylation is provided as a food and beverage composition, the oral composition for promoting DNA methylation may be offered or sold as a food or beverage with an indication of the application for promoting DNA methylation. The application includes health purposes. The act of "indication" includes all acts that inform consumers of the aforementioned application. Any expression that evokes or suggests the aforementioned application is considered an act of "indication", regardless of the purpose, content, object, medium, and the like of the indication.

The "indication" is preferably made in a manner that allows the consumers to directly recognize the aforementioned application. Specifically, this includes acts such as an act of transferring, delivering, exhibiting for transfer or delivery, or importing food and beverage products or product packaging with the aforementioned application described; and an act of exhibiting or distributing advertisements, price lists, or transaction documents relating to the products with the aforementioned application described, or providing information of the content with the aforementioned application described via electromagnetic means such as the Internet.

The "indication" may include indications as health foods, functional foods, enteral nutritional foods, foods for special uses, foods with health claims, foods for specified health uses, foods with nutrient function claims, foods with function claims, quasi-drugs, and the like. Among these, particularly, indications approved under systems for foods for specified health uses, foods with nutrient function claims, or foods with function claims, or similar systems are included. Specifically, examples of the indications include an indication as foods for specified health uses, an indication as conditional foods for specified health uses, an indication of effects on body structure or function, an indication for disease risk reduction, and an indication of functionality based on scientific evidence. More specifically, typical examples thereof include an indication as foods for specified health uses, in particular, an indication of health purposes, and similar indications.

In a further aspect of the present embodiment, use of the compound or the like for producing the DNA methylation promoter or the oral composition for promoting DNA methylation is also provided. In yet another aspect of the present embodiment, a method of promoting DNA methylation including a step of administering the compound or the like to a subject is provided. In still another aspect of the present embodiment, the compound or the like for the use in promoting DNA methylation is provided.

The present disclosure is more specifically described with reference to the following Examples, but the present disclosure is not limited to the Examples.

### Examples

### Example 1: Synthesis of Compound KIT-13

KIT-13 was synthesized as a compound according to Formula (I) as follows.

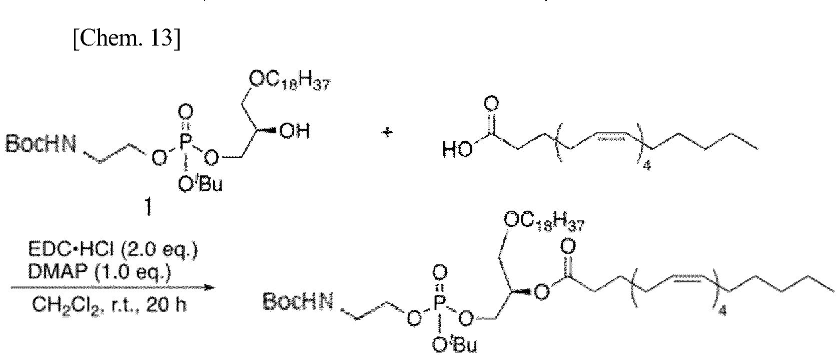

To a dichloromethane solution (3 mL) of compound 1 (tert-butyl(2-((tert-butoxy((R)-2-hydroxy-3-(octadecyloxy)propoxy)phosphoryl)oxy)ethy l)carbamate (187 mg, 0.30 mmol), EDC hydrochloride (115 mg, 0.60 mmol), DMAP (37 mg, 0.30 mmol), and arachidonic acid (100 mg, 0.33 mmol) were added at room temperature, and the resulting mixture was stirred for 20 hours at room temperature. The reaction solution was diluted with ethyl acetate, and the resulting dilution was washed with water. The ethyl acetate layer was dried over anhydrous sodium sulfate, and then concentrated under reduced pressure. Purification of the residue was performed by column chromatography to obtain a desired product, (2R)-1-((tert-butoxy(2-((tert-butoxycarbonyl)amino)ethoxy)phosphoryl)oxy)-3-(octadecy loxy)propan-2-yl(5Z,8Z,11Z,14Z)-icosa-5,8,11,14-tetraenoate, as a colorless liquid (260 mg, 95%).

¹H NMR (500 MHz, CDCl₃) δ 5.43-5.31 (m, 8H), 5.16 (quin, J = 4.9 Hz, 1H), 5.16 (br, 1H), 4.21 (m, 1H), 4.14-4.09 (m, 1H), 4.07-4.02 (m, 2H), 3.55 (d, J = 5.3 Hz, 2H), 3.47-3.38 (m, 5.3 Hz), 2.85-2.79 (m, 6H), 2.35 (t, J = 7.6 Hz), 2.12 (q, J = 7.0 Hz, 2H), 2.06 (q, J = 7.1 Hz, 2H), 1.74-1.67 (m, 2H), 1.56-1.54 (m, 2H), 1.502 (s, 9H, one diastereomer), 1.496 (s, 9H, the other diastereomer), 1.39-1.25 (m, 38H), 0.90-0.87 (m, 6H).

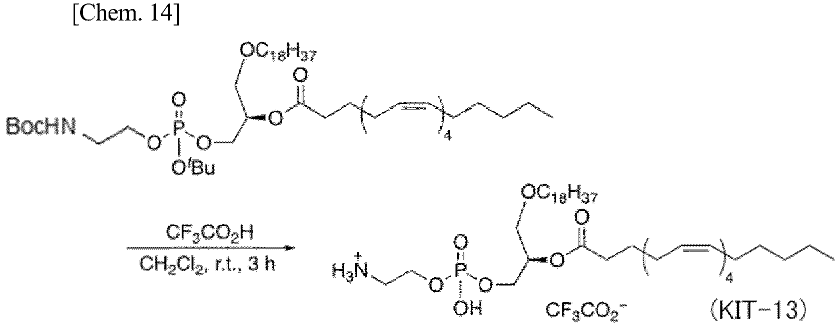

To a dichloromethane solution (2 mL) of (2R)-1-((tert-butoxy(2-((tert-butoxycarbonyl)amino)ethoxy)phosphoryl)oxy)-3-(octadecy loxy)propan-2-yl(5Z,8Z,11Z,14Z)-icosa-5,8,11,14-tetraenoate (260 mg, 0.29 mmol), 2 mL of trifluoroacetic acid was added at room temperature, and the resulting mixture was stirred for 3 hours at room temperature. The reaction mixture was concentrated under reduced pressure, to obtain a desired product, 2-(hydroxy((R)-2-(((5Z,8Z,11Z,14Z)-icosa-5,8,11,14-tetraenoyl)oxy)-3-(octadedecyloxy) propoxy)phosphoryl)oxy)ethane-1-ammonium 2,2,2-trifluoroacetate (KIT-13), as a colorless liquid (249 mg, quant.).

KIT-13: ¹H NMR (500 MHz, CDCl₃) δ 11.31 (br, 1H), 5.41-5.31 (m, 8H), 5.16-5.15 (m, 1H), 4.17 (br, 2H), 4.07-4.00 (m, 2H), 3.54 (d, J = 4.9 Hz, 2H), 3.45-3.39 (m, 2H), 3.27 (br), 2.84-2.78 (m, 6H), 2.35 (t, J = 7.4 Hz, 2H), 2.10 (q. J = 7.1 Hz, 2H), 2.05 (q. J = 7.2 Hz, 2H), 1.69-1.66 (m, 2H), 1.54-1.51 (m, 2H), 1.39-1.25 (m, 38H), 0.90-0.86 (m, 6H).

### Example 2: Synthesis of Compound KIT-18

KIT-18 was synthesized as a compound according to Formula (I) as follows.

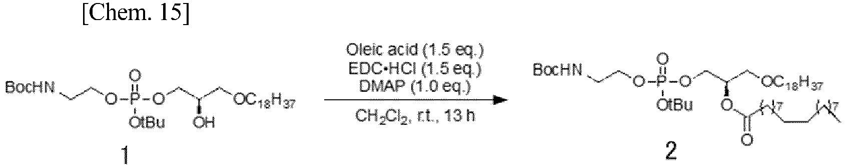

To a CH₂Cl₂ (8.0 ml) solution of oleic acid (338 mg, 1.20 mmol), DMAP (98 mg, 0.80 mmol), and compound 1 (500 mg, 0.80 mmol), 1-(3-dimethylaminopropyl)-3-ethylcarbodiimide hydrochloride (EDC·HCl, 230 mg, 1.20 mmol) was added at 0°C, and the resulting mixture was stirred at room temperature for 13 hours. The reaction mixture was then diluted with ethyl acetate (40 ml), and the resulting dilution was washed with water. After drying with Na₂SO₄, filtration and concentration were performed, and then purification of the resultant was performed by column chromatography (hexane/ethyl acetate = 1/2) to obtain a desired product, compound 2 ((2R)-1-((tert-butoxy(2-((tert-butoxycarbonyl)amino)ethoxy)phosphoryl)oxy)-3-(octadec yloxy)propan-2-yl oleate), as a clear liquid in 42% yield (300 mg, 0.338 mmol).

Compound 2: ¹H NMR (500 MHz, CDCl_{3,} a mixture of diastereomers) d 5.38-5.30 (2H, m), 5.18-5.12 (2H, m), 4.21-4.15 (1H, m), 4.15-4.09 (1H, m), 4.08-4.02 (2H, m), 3.55 (2H, d, J = 5.2 Hz), 3.47-3.37 (4H, m), 2.33 (2H, t, J = 7.6 Hz), 2.03-1.98 (4H, m), 1.65-1.59 (2H, m), 1.57-1.51 (2H, m), 1.50 (9H, d, J = 3.4 Hz), 1.44 (9H, s), 1.34-1.23 (50H, m), 0.88 (3H, t, J = 6.9 Hz), 0.88 (3H, t, J = 6.9 Hz).

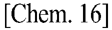

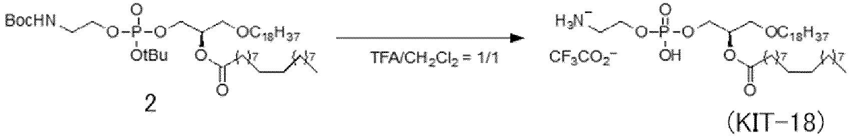

To compound 2 (300 mg, 0.338 mmol), TFA (3 ml) and CH₂Cl₂ (3 ml) were added, and the resulting mixture was stirred at room temperature for 3 hours. The reaction mixture was concentrated under reduced pressure, to quantitatively obtain a desired product, KIT-18 (2-((hydroxy((R)-3-(octadecyloxy)-2-(oleoyloxy)propoxy)phosphoryl)oxy)ethane-1 aminium trifluoroacetate) as a yellow liquid (258 mg, 0.338 mmol).

KIT-18: ¹H NMR (500 MHz, CDCl₃) d 10.42 (1H, brs), 8.05 (3H, brs), 5.38-5.30 (2H, m), 5.17-5.12 (1H, m), 4.18-4.10 (2H, m), 4.07-3.95 (2H, m), 3.53 (2H, d, J = 4.8 Hz), 3.47-3.39 (2H, m), 3.27-3.18 (2H, m), 2.32 (2H, t, J = 7.6 Hz), 2.04-1.98 (4H, m), 1.64-1.49 (4H, m), 1.34-1.23 (50H, m), 0.88 (3H, t, J = 6.9 Hz), 0.88 (3H, t, J = 6.9 Hz).

### Example 3: Synthesis of Compound KIT-24

KIT-24 was synthesized as a compound according to Formula (I) as follows.

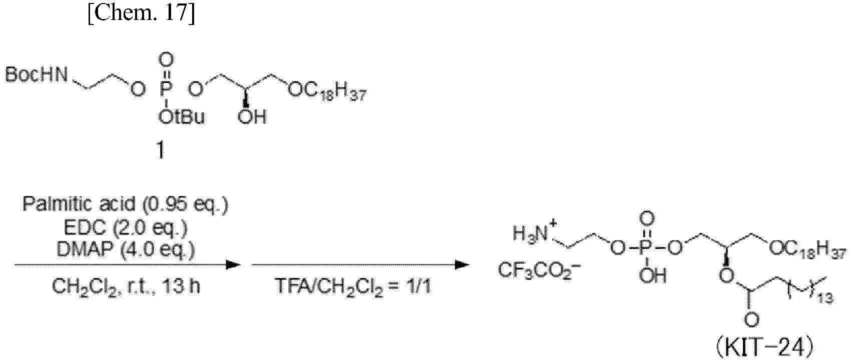

To a CH₂Cl₂ (1.6 ml) solution of palmitic acid (38 mg, 0.15 mmol), DMAP (78 mg, 0.64 mmol), and compound 1 (100 mg, 0.16 mmol), 1-(3-dimethylaminopropyl)-3-ethylcarbodiimide (EDC, 0.06 mL, 0.32 mmol) was added at 0°C, and the resulting mixture was stirred at room temperature for 13 hours. The reaction mixture was then diluted with ethyl acetate (10 ml), and the resulting dilution was washed with water. After drying with Na₂SO4, filtration and concentration were performed, and then crude purification of the resultant was performed by column chromatography (hexane/ethyl acetate = 1/2). To the obtained clear liquid, TFA (1 ml) and CH₂Cl₂ (1 mL) were added and the mixture was stirred at room temperature for 3 hours. The reaction mixture was concentrated and purified by column chromatography (CHCl₃/MeOH = 5/1). Thereafter, TFA (1 ml) and CH₂Cl₂ (1 ml) were added again and the mixture was stirred for 1 hour, to obtain a desired product, KIT-24 (2-((hydroxy((R)-3-(octadecyloxy)-2-(palmitoyloxy)propoxy)phosphoryl)oxy)ethane-1 aminium trifluoroacetate), as a yellow liquid in 98% yield (40.6 mg, 0.057 mmol).

KIT-24: ¹H NMR (500 MHz, CDCl₃) d 7.62 (3H, brs), 5.19-5.15 (1H, m), 4.32-3.94 (4H, m), 3.59-3.28 (6H, m), 2.35-2.29 (2H, m), 1.60-1.50 (4H, m), 1.32-1.22 (54H, m), 0.88 (3H, t, J = 7.0 Hz), 0.88 (3H, t, J = 7.0 Hz).

### Test Example 1

In the following, a vector that expresses shRNA targeting mouse MeCP2 (NM_010788) (catalog number: TRCN0000304464, manufactured by Sigma) was used as "shMeCP2 vector". An amplification product from shMeCP2 vector was obtained by PCR to prepare a vector excluding the MeCP2-targeting sequence. The vector was designated as "shCont. vector". The PCR was carried out using a forward primer and a reverse primer for shMeCP2 vector having the base sequences of SEQ ID NO:1 and SEQ ID NO:2, respectively.

Mouse glial cell derived BV2 cells cultured in Dulbecco's Modified Eagle Medium (DMEM, manufactured by Sigma-Aldrich) supplemented with 10% fetal calf serum (FCS, manufactured by Sigma-Aldrich) were transfected with either the shMeCP2 vector or the shCont. vector. By utilization of the puromycin resistance gene contained in the vector, puromycin-resistant lines (concentration of puromycin, 1 µg/ml) were separated. The resulting cells were designated as BV2/shMeCP2 and BV2/shCont., respectively.

BV2/shMeCP2 was cultured for 2 days in DMEM (manufactured by Sigma-Aldrich) containing 10% FCS (manufactured by Sigma-Aldrich), 100 units/ml penicillin, and 100 µg/ml streptomycin (manufactured by Nacalai) in the presence of 5 µg/ml KIT-13 (shMeCP2 + KIT-13) or in the absence of KIT-13 (shMeCP2). As a control, BV2/shCont. was cultured for 2 days in the same medium in the absence of KIT-13 (shCont.).

Cells were fixed with a 4% paraformaldehyde (PFA) solution and washed with phosphate-buffered saline (PBS). The obtained samples were treated with 2N HCl at 37°C for 15 minutes. The samples were washed with PBS, and treated with 1% Tx-100/PBS at room temperature for 2 minutes. After washing with PBS, the samples were treated with 1% bovine serum albumin (BSA). An anti-5-mC antibody (#28692, manufactured by Cell Signaling technology) was diluted 1:4000 in 1% BSA-PBS and used as a primary antibody. The primary antibody was reacted with the samples at 25°C for 2 hours. After washing the samples with PBS, the samples were reacted at room temperature for 1 hour in the dark with a solution in which Alexa Fluor 488 anti-rabbit IgG (manufactured by Invitrogen) and 3 mM DAPI were diluted 1:2000 in 1% BSA-PBS. The samples were washed with PBS and rinsed with dH₂O, followed by embedding with a cover glass and observation with BZ-X810 (manufactured by Keyence).

### Results

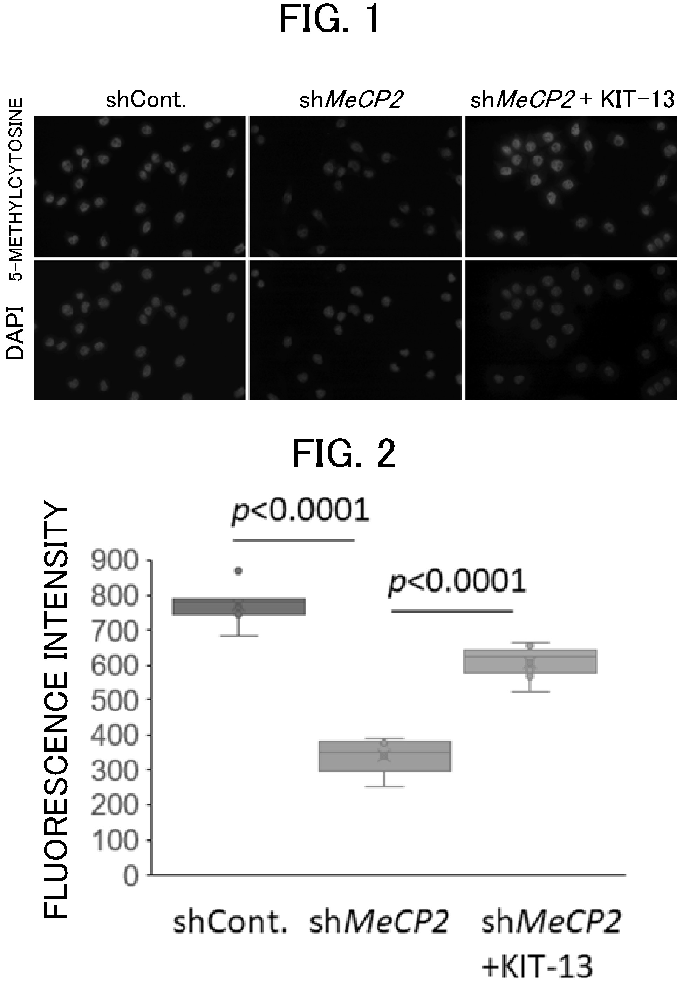

Fluorescent staining images of the samples are shown in FIG. 1. Fluorescence intensity quantified based on the fluorescent staining images is shown in FIG. 2. In shMeCP2, fluorescence intensity derived from 5-methylcytosine decreased compared with that of shCont. In shMeCP2 + KIT-13, fluorescence intensity derived from 5-methylcytosine significantly increased compared with that of shMeCP2.

### Test Example 2

TIG-107 cells (JCRB0532) and TIG-120 cells (JCRB0542) were obtained from the JCRB Cell Bank. TIG-107 cells were cultured for 4 days in DMEM (manufactured by Sigma-Aldrich) containing 10% FCS (manufactured by Sigma-Aldrich), 100 units/ml penicillin, and 100 µg/ml streptomycin (manufactured by Nacalai) in the presence of 10 µg/ml KIT-13 (TIG-107 + KIT-13) or in the absence of KIT-13 (TIG-107). As a control, TIG-120 cells were cultured for 4 days in the same medium in the absence of KIT-13 (TIG-120). As in Test Example 1, fluorescent staining images of the samples prepared from the cultured cells were observed.

### Results

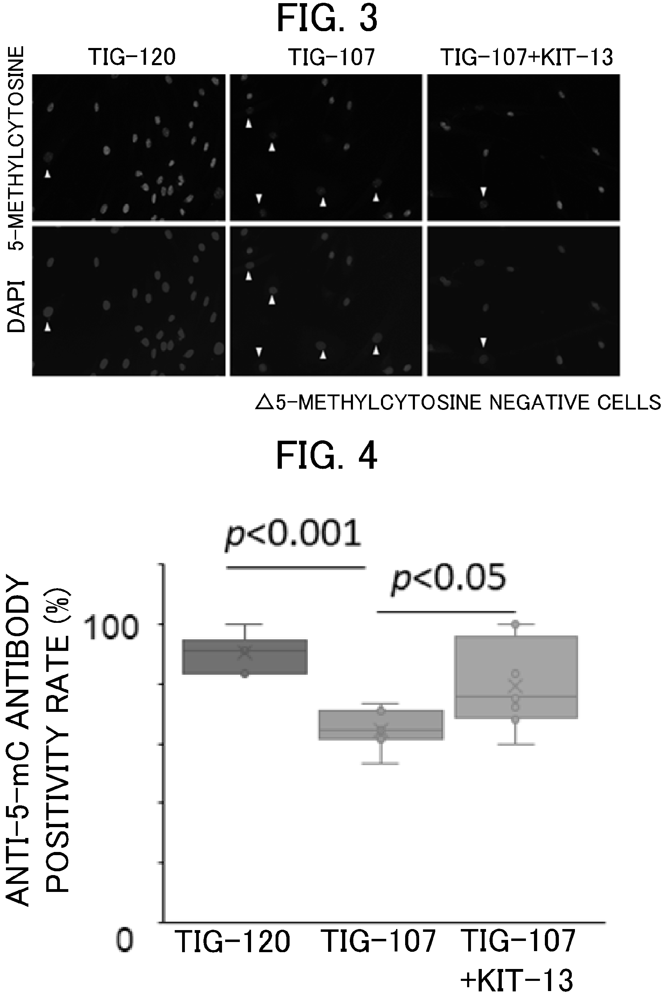

The fluorescent staining images of the samples are shown in FIG. 3. Anti-5-mC antibody positivity rates calculated from the fluorescent staining images are shown in FIG. 4. In TIG-107 + KIT-13, cells positive for 5-methylcytosine significantly increased compared cells cultured with TIG-107.

### Test Example 3

TIG-107 cells were cultured for 4 days in DMEM (manufactured by Sigma-Aldrich) containing 10% FCS (manufactured by Sigma-Aldrich), 100 units/ml penicillin, and 100 µg/ml streptomycin (manufactured by Nacalai) in the presence of 10 µg/ml KIT-18, in the presence of 10 µg/ml KIT-24 or in the absence of these (-). As in Test Example 1, fluorescent staining images of the samples prepared from the cultured cells were observed, and anti-5-mC antibody positivity rates were determined from the fluorescent staining images.

### Results

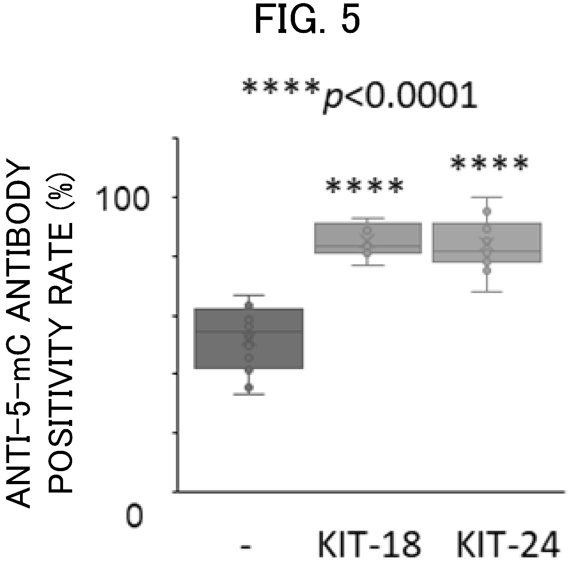

Anti-5-mC antibody positivity rates calculated from the fluorescent staining images of the samples are shown in FIG. 5. KIT-18 or KIT-24 significantly increased cells positive for 5-methylcytosine.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2024-009152, filed on January 25, 2024, the entire disclosure of which is incorporated by reference herein.

### Industrial Applicability

The present disclosure is useful for promoting or inducing methylation of genomic DNA.

### Sequence Listing

24F129-PCT.xml

## Claims

1. A DNA methylation promoter, comprising:
a compound of Formula (I), a racemate of the compound, or a salt of the compound or the racemate, 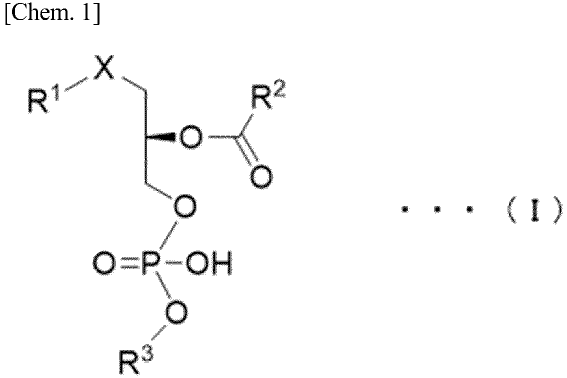 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 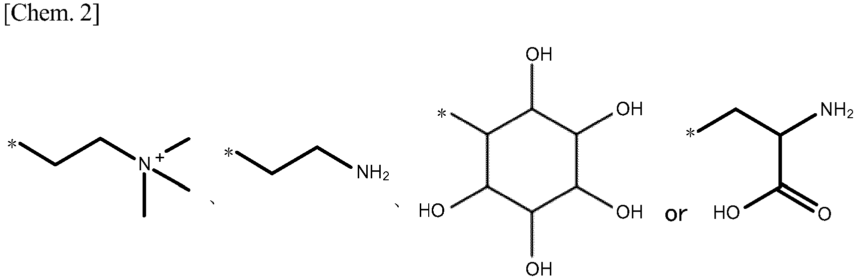 optionally having one or more substituents, in which * is an oxygen atom bound to R³).

2. An oral composition for promoting DNA methylation, the oral composition comprising:
a compound of Formula (I), a racemate of the compound, or a salt of the compound or the racemate, 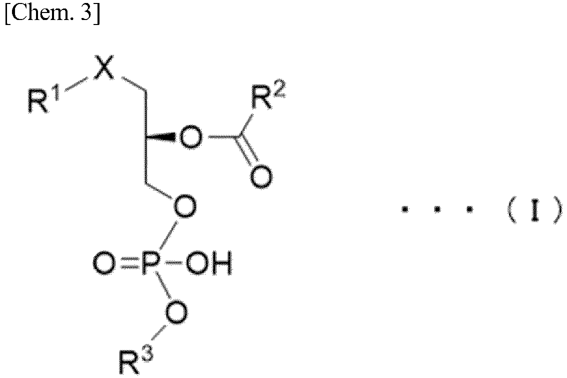 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 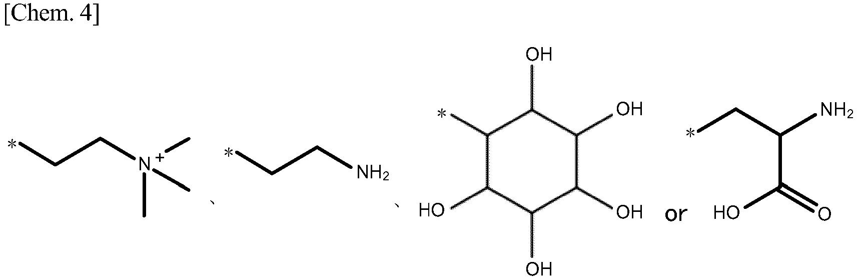 optionally having one or more substituents, in which * is an oxygen atom bound to R³).

3. The DNA methylation promoter according to claim 1 or the oral composition according to claim 2, wherein
X represents an oxygen atom, and R¹ represents an alkyl group having 18 carbon atoms.

4. The DNA methylation promoter or the oral composition according to claim 3, wherein
R², when defined as R²COOH, represents a monovalent group corresponding to arachidonic acid, oleic acid, or palmitic acid.

5. Use of a compound of Formula (I), a racemate of the compound, or a salt of the compound or the racemate, for producing a DNA methylation promoter or an oral composition for promoting DNA methylation, 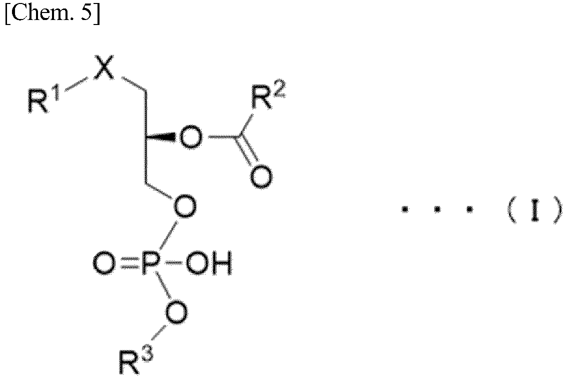 (where, in Formula (I), X represents a carbon atom or an oxygen atom, R¹ represents a saturated aliphatic hydrocarbon group optionally containing a ring structure, R² represents a saturated or unsaturated aliphatic hydrocarbon group having 10 to 30 carbon atoms, optionally containing one or more substituents that are each an alkyl group having 1 to 4 carbon atoms or an alkoxy group having 1 to 4 carbon atoms, each carbon atom of a glycerol backbone optionally has one or more substituents, and R³ is 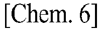 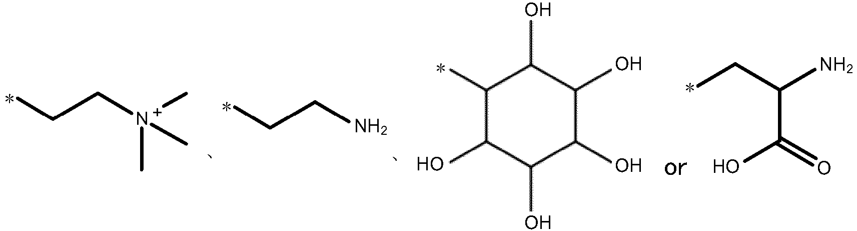 optionally having one or more substituents, in which * is an oxygen atom bound to R³).
